# EUROPEAN PATENT SPECIFICATION

(11) **EP 1 936 377 B1**
(45) Date of publication and mention of the grant of the patent: **26.02.2014**
(21) Application number: 07122883.7
(22) Date of filing: 11.12.2007
(51) Int. Cl.: G01N 33/542, G01N 33/573

(54) **Method for detection of cAMP and cGMP**
Verfahren zur Erkennung von cAMP und cGMP
Procédé pour la détection de cAMP et de cGMP

(30) Priority: 21.12.2006 EP 06126927
(43) Date of publication of application: 25.06.2008
(73) Proprietor: F. Hoffmann-La Roche AG, 4070 Basel (CH)
(72) Inventor: Enderle, Thilo, 79618 Rheinfelden (DE); Matile, Hugues, 4001 Basel (CH); Roth, Doris, 4051 Basel (CH)
(74) Representative: Küng, Peter

(56) References cited:
- KRAEMER A ET AL: "Dynamic interaction of cAMP with the rap guanine-nucleotide exchange factor epac1" JOURNAL OF MOLECULAR BIOLOGY, LONDON, GB, vol. 306, no. 5, 9 March 2001 (2001-03-09), pages 1167-1177, XP004486194 ISSN: 0022-2836
- KRAEMER A: "Untersuchung zur Interaktion von humanem Ras und Rap mit regulatorischen Proteinen"[Online] 2001, pages 1-129, XP002472943 Bochum Retrieved from the Internet: URL:http://deposit.ddb.de/cgi-bin/dokserv? idn=963978993&dok_var=d1&dok_ext=pdf&filen ame=963978993.pdf> [retrieved on 2008-03-13]
- GABRIEL DANIELA ET AL: "High throughput screening technologies for direct cyclic AMP measurement." April 2003 (2003-04), ASSAY AND DRUG DEVELOPMENT TECHNOLOGIES APR 2003, VOL. 1, NR. 2, PAGE(S) 291 - 303 , XP002472944 ISSN: 1540-658X * page 293, left-hand column, paragraph 3 - page 295, left-hand column, paragraph 2 *
- CAMPBELL A K ET AL: "A HOMOGENEOUS IMMUNOASSAY FOR CYCLIC NUCLEOTIDES BASED ON CHEMILUMINESCENCE ENERGY TRANSFER" BIOCHEMICAL JOURNAL, PORTLAND PRESS, LONDON, GB, vol. 216, no. 1, 1983, pages 185-194, XP009022777 ISSN: 0264-6021
- MOLL D ET AL: "Biomolecular interaction analysis in functional proteomics" August 2006 (2006-08), JOURNAL OF NEURAL TRANSMISSION, VOL. 113, NR. 8, PAGE(S) 1015-1032 , XP002472945 ISSN: 0300-9564 * the whole document *
- WILLIAMS C: "CAMP DETECTION METHODS IN HTS: SELECTING THE BEST FROM THE REST" NATURE REVIEWS. DRUG DISCOVERY, NATURE PUBLISHING GROUP, BASINGSTOKE, GB, vol. 3, no. 2, February 2004 (2004-02), pages 125-135, XP008032131 ISSN: 1474-1784
- THOMSEN ET AL: "Functional assays for screening GPCR targets" CURRENT OPINION IN BIOTECHNOLOGY, LONDON, GB, vol. 16, no. 6, December 2005 (2005-12), pages 655-665, XP005173499 ISSN: 0958-1669

## Description

Nucleotides play an important role in the signal transduction of a cell. For example, the nucleotide cAMP is involved in the signal transduction of G-protein-coupled receptors (GPCRs).

GPCRs form the largest class of pharmaceutical drug targets. In order to test the activity of potential new medicines on GPCRs in drug discovery, assays are needed which monitor the function of the receptors in their cellular environment. For high throughput screening, very early in the drug discovery process, these functional assays need to be simple with a low number of steps, sensitive to detect minute effects of early compounds and they need to comprise a robust readout to be applied in an automated fashion.

Functional assays for GPCRs can be set-up by detection of second messenger molecules reflecting the activation state of the receptor. Such a second messenger molecule is cyclic adenosine monophosphate (cAMP) formed upon modulation of the adenylyl cyclase activity. An overview on commercially available, functional assay kits that determine the level of cellular cAMP based on fluorescence or chemiluminescence readout is given by W. Thomsen et al. (Current Opinion in Biotechnology 2005, 16, 655-665).

Kraemer et al. investigate in Journal of Molecular Biology 2001, 306, 1167-1177 the equilibrium and kinetics of the interaction of cAMP and Epac1 utilizing fluorescent cAMP analogues, and different domain constructs of Epac1.

Gabriel et al. disclose in Assay and Drug Development Technologies, 2003, 1, 291-303 fluorescence based bioassays for the detection of receptor activity and ligand effects in cells expressing the receptors of the respective signal transduction pathways by determining cAMP in cells with or without ligand treatment using lysis.

Williams, C. reviews in Nature Review, Drug Discovery, 2004, 3, 125-135 cAMP detection methods.

The present invention provides a simple assay for detecting cAMP and its use for indirectly detecting receptor activity and for screening of ligands.

This invention is based on the surprising fact that conjugates with cAMP derivates designed by modification of the nucleobase may quench the fluorescence emission of a fluorophore while cAMP does not quench the fluorophore.

Therefore, the present invention provides an *in vitro* method for detecting cyclic adenosine monophosphate (cAMP) or cyclic guanosine monophosphate (cGMP) comprising a) contacting a mixture comprising cAMP or cGMP with a complex of a tracer and a dequencher, wherein the tracer is a fluorophore covalently linked to a cAMP quencher, the cAMP quencher is 2-(6-Aminohexyl)amino-cAMP, 8-(6-Aminohexyl)amino-cAMP, 8-(8-Amino-3, 6-dioxaoctylamino)-cAMP, 8-Hydroxy-cAMP or 8-(4-Mercaptobutylthio)-cAMP, and the cAMP quencher is able to quench the fluorophore it is covalently linked to, and the dequencher is able to bind the cAMP quencher as well as the cyclic nucleotide which shall be detected, and b) measuring the change in fluorescence, wherein the measured change of the fluorescence is compared with a control.

Surprisingly, the assay may also be used to detect cGMP in a mixture. Unlike cAMP cGMP has quenching capabilities, but only in a concentration of about 1mM and above. However, cellular cGMP concentrations are usually in the range of several orders of magnitude below 1mM.

Therefore, the present invention provides an in vitro method for detecting cAMP or cGMP.

The measured change of the fluorescence is compared with a control. The control may be a standard curve with predetermined amount of nucleotide.

The term "mixture" refers to two or more substances mixed together in such a way that each remains unchanged. The mixture includes but is not restricted to cell lysates, cell culture supernatants, biological fluids such as serum, plasma, urine, bronchial lavage fluid, sputum, biopsies like cerebrospinal fluid.

The term "nucleotide" refers to a molecule comprising a nucleobase, a sugar, and one (MP), two (DP) or three (TP) phosphate groups. There are five nucleobases: Adenine (A), Uracil (U), Thymine (T), Guanine (G) and Cytosine (C). The sugar is either a ribose or a desoxyribose (d).

The term "cyclic nucleotide" refers to a nucleotide in which the phosphate group is bonded to two of the sugar's hydroxyl groups, forming a cyclical or ring structure. These include cyclic AMP (cAMP), cyclic CMP (cCMP), cyclic TMP (cTMP), cyclic UMP (cUMP) and cyclic GMP (cGMP).

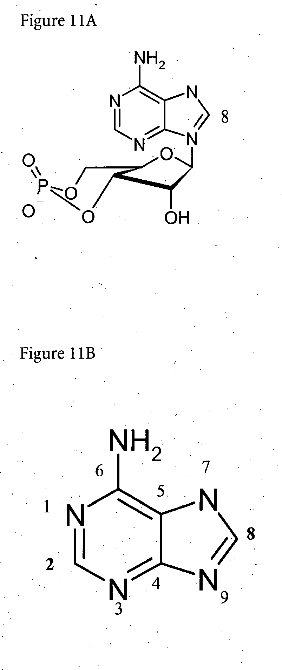

The term "cAMP quencher" as used herein refers to a derivate of cAMP which is able to quench the fluorophore used in the assay. The quencher is covalently linked to said fluorophore. The cAMP quencher is a derivate of cAMP whereby the nucleobase is substituted with an electron donor. The position of the nucleobase for a substitution is position 2 or 8 as shown in Figure 11B. Derivates of cAMP are 2-(6-Aminohexyl)amino-cAMP (2-AHA-cAMP), 8-(6-Aminohexyl)amino-cAMP (8-AHA-cAMP), 8-(8-Amino-3, 6-dioxaoctylamino)-cAMP (8-ADOA-cAMP), 8-Hydroxy-cAMP (8-OH-cAMP), 8-(4-Mercaptobutylthio)-cAMP (8-MBT-cAMP). The most preferred derivative is 2-AHA-cAMP.

The term "fluorophore" refers to a molecule emitting fluorescence when excited with a specific wavelength and being statically quenchable. A preferred fluorophore is an oxazine derivate as described in EP 747 447 such as for example MR121, Evoblue30 or JA314. Further preferred fluorophores which may be used are ATTO™ 590, ATTO™ 655, ATTO™ 680, Atto™ 700 (Atto-Tec GmbH, Am Eichenhang 50, 57076 Siegen, Germany). More preferably, the fluorophore is MR121 or Atto™ 700.

The term "dequencher" as used herein refers to a molecule which reverses the quenching effect of the cAMP quencher by binding to the cAMP quencher. It is essential that the dequencher is able to bind the cAMP quencher as well as the cyclic nucleotide which shall be detected i.e. cAMP or cGMP.

The dequencher may be a binding protein. Preferably, the dequencher is a cAMP-specific antibody. The antibody may be a monoclonal or a polyclonal antibody. If the dequencher is a protein, the pH value has to be adapted to a range in which the protein is capable of binding. Normally, this range is pH 6.8 to 7.8.

Methods for producing antibody which are specific for a nucleotide are well known to the skilled in the art. Kohler and Milstein (Nature 1975, 256: 495-497), for example, describe methods for producing monoclonal antibodies.

The detection complex comprises a tracer and a dequencher. The tracer consists of a fluorophore covalently linked to a cAMP quencher. The detection complex shows fluorescence if excited. When the detection complex is contacted with a nucleotide, the dequencher binds a certain percentage of the free nucleotide and leaves the detection complex. The tracer is now not anymore dequenched and shows only low or no fluorescence when excited. This decrease of the fluorescence is indicative for the amount of nucleotide present in the system.

The change of fluorescence may be compared with a control such as a standard curve. A standard curve may be established by measuring the change in fluorescence for predetermined amounts of the nucleotide which shall be detected.

The present invention further provides the use of the methods as described above for determining the cAMP concentration in a mixture. Furthermore, the methods as described above may be used for determining the activity of receptors wherein the signal transduction of these receptors comprises cAMP or cGMP. Preferably, the receptor is a G-protein coupled receptor (GPCR).

Therefore, the present invention also provides an *in vitro* method for determining the activity of a receptor, wherein the signal transduction of the receptor comprises cAMP or cGMP a) contacting cells expressing said receptor with a complex of a tracer and a dequencher, wherein the tracer is a fluorophore covalently linked to a cAMP quencher, the cAMP quencher is 2-(6-Aminohexyl)amino-cAMP, 8-(6-Aminohexyl)amino-cAMP, 8- (8-Amino-3, 6-dioxaoctylamino)-cAMP, 8-Hydroxy-cAMP or 8-(4-Mercaptobutylthio)-cAMP, and the cAMP quencher is able to quench the fluorophore it is covalently linked to, and the dequencher is able to bind the cAMP quencher as well as the cyclic nucleotide which shall be detected, b) lysing the cells, and c) measuring the change in fluorescence, wherein the measured change of the fluorescence is compared with a control.

Alternatively, step b) may be prior of step a).

A preferred embodiment is a method for determining the activity of a GPCR.

Furthermore, the present invention provides the use of the methods as described above for screening ligands of a receptor wherein the signal transduction of this receptor comprises cAMP or cGMP. Preferably, said receptor is a GPCR.

Therefore, the present invention also provides an in *vitro* method for screening of a ligand for a receptor, wherein the signal transduction of the receptor comprises cAMP or cGMP comprising a) contacting a candidate compound with a cell expressing said receptor, b) adding to the cells a complex of a tracer and a dequencher, wherein the tracer is a fluorophore covalently linked to a cAMP quencher, the cAMP quencher is 2-(6-Aminohexyl)amino-cAMP, 8-(6-Aminohexyl)amino-cAMP, 8-(8-Amino-3, 6-dioxaoctylamino)-cAMP, 8-Hydroxy-cAMP or 8-(4-Mercaptobutylthio)-cAMP, and the cAMP quencher is able to quench the fluorophore it is covalently linked to, and the dequencher is able to bind the cAMP quencher as well as the cyclic nucleotide which shall be detected, c) lysing cells, and d) measuring the change in fluorescence, wherein the measured change of the fluorescence is compared with a control.

Alternatively, step c) may be prior of step b).

A preferred embodiment is a method for screening a ligand for a GPCR.

The measured change of the fluorescence is compared with a control. The control may be a standard curve with predetermined amount of nucleotide.

A cell expressing a receptor such as for example GPCR may be a cell which expresses said receptor endogenously or a cell transgenic for said receptor.

Such transgenic cell may be created by methods known in the art. A preferred method comprises the following steps: cloning a DNA which encodes the receptor of interest, inserting said DNA into a vector and introducing said vector into the cell. Preferably, said vector comprises stretches which are homologous to stretches in the genome of the cell suitable for homologous recombination and thereby allowing the targeted insertion of the DNA encoding the receptor into the genome of the cell.

The term "ligand" as used herein refers to a molecule that binds to a receptor. A ligand may be an agonist, an antagonist, a modulator, partial agonist or a partial antagonist.

The term "agonist" refers to a molecule that binds to a receptor and triggers a response in the cell. The term "partial agonist" refers to a molecule that partially activates a receptor. The term "antagonist" refers to a molecule that binds to the receptor but fails to activate the receptor and actually blocks it from activation by an agonist.

The present invention also relates to a kit comprising a dequencher and a fluorophore covalently linked with a cAMP quencher as described above. The kit may comprise the dequencher and the modified fluorophore separately or in a complex.

The kit may also comprise any other components deemed appropriate in the context of measuring the level(s) of the respective fluorophores, such as suitable buffers, filters, etc.

Optionally, the kit may additionally comprise a user's manual for interpreting the results of any measurement(s) with respect to determining the GPCR activity. Particularly, such manual may include information for the interpretation of the measured change in fluorescence, preferably a standard curve.

Having now generally described this invention, the same will become better understood by reference to the specific examples, which are included herein for purpose of illustration only and are not intended to be limiting unless otherwise specified, in connection with the following figures.

### Figures

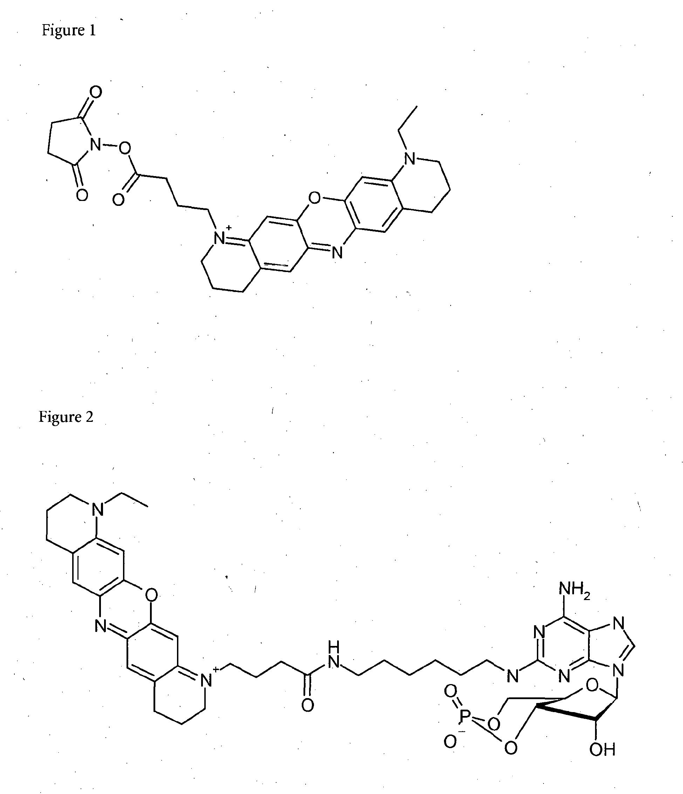

Figure 1 shows the molecular structure of the fluorophore MR121 as NHS ester as it was used for labeling of the cAMP derivative in the examples.
Figure 2 shows the molecular structure of MR121-2-AHA-cAMP.

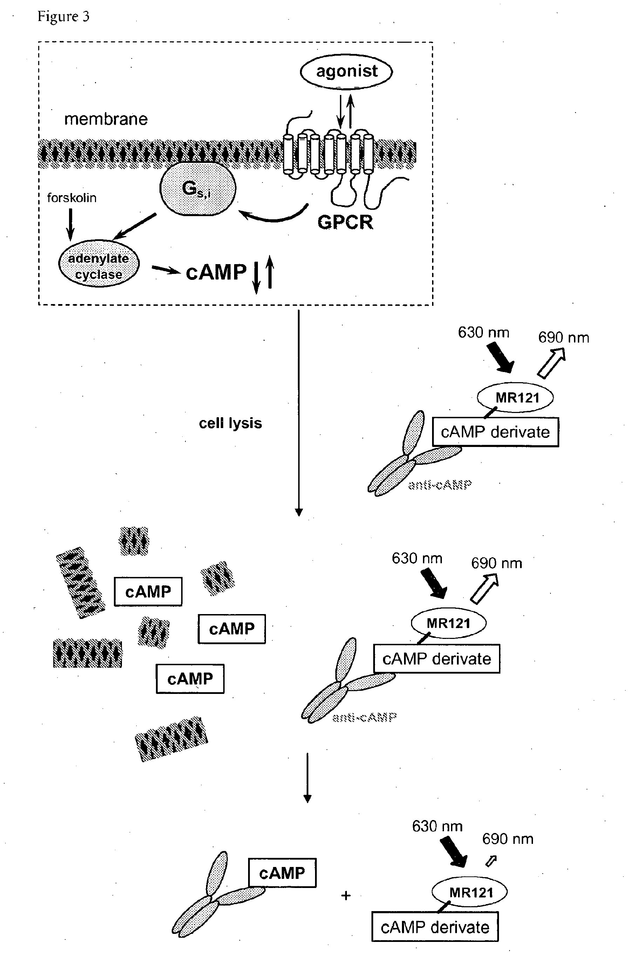

Figure 3 shows a schematic representation of the assay principle with the example of determining cAMP concentration in a cell. The cells are lysed and a detection mix is added to the cell lysates. The detection mix comprises a complex of a fluorophore (e.g. MR121) linked to a cAMP quencher (cAMP derivate, e.g. 2-AHA-cAMP) and cAMP antibody (e.g. mouse monoclonal anti-cAMP antibody). The quenching effect of the quencher on the fluorophore is revoked by antibody (= dequencher). When contacted with cAMP, the antibody binds it instead of the cAMP derivate. Without the dequenching effect of the antibody, the cAMP quencher quenches the fluorophore and causes a decrease of fluorescent signal. The higher the cAMP concentration, the lower is the fluorescence.
Figure 4 shows a graphical representation of Stern-Volmer-Plots of MR121 and Tryptophane (a), 2-AHA-cAMP (b) and cAMP (c). I₀/I: change in fluorescence intensity ( I₀ fluorescence intensity in the absence and I in the presence of a quencher), to/t: change of lifetime of the fluorescence (to fluorescence lifetime in the absence and t in the presence of a quencher).
Figure 5 shows a graphical representation of the decrease of fluorescence intensity (I₀/I) for different cAMP derivatives (8-AHA-cAMP, 2-AHA-cAMP, N-6-Aminohexyl-cAMP, 8-OH-cAMP, 8-ADOA-cAMP, 2'-AEC-cAMP, 8-MBT-cAMP). The large deviation from linearity for 8-AHA-cAMP above -5 mM is due to solubility problems. I₀/I: change in fluorescence intensity
Figure 6: shows a graphical representation of the quenching and dequenching of the fluorescence of the tracer MR121-2-AHA-cAMP using two different specific cAMP antibodies A and B. Ab: Antibody
Figure 7 shows a graphical representation of the titration of the anti-cAMP antibody at a fixed tracer concentration (10 nM MR121-2-AHA-cAMP). A fit of the experimental data resulted in a K_{d} value of 28 nM.
Figure 8 shows a graphical representation of two cAMP standard curves, one without cells (filled diamonds) and one in the presence of cells (open diamonds), fitting of the curves gives IC₅₀=109 nM and 119 nM without and with cells, respectively.
Figure 9 shows a graphical representation of a Forskolin dose response curve and a cAMP standard curve with 10'000 cells/well, fitting of the curves gives IC₅₀=102 nM for cAMP and 3.7 µM for forskolin.
Figure 10 shows a graphical representation of Stern-Vollmer Plots of the following molecules: MR121-8-AHA-cAMP, ATTO590-8-AHA-cAMP and ATTO655-8-AHA-cAMP.
Figure 11 shows the chemical structure of cyclic adenosine monophosphate (cAMP) (A) and the purine ring of cAMP (B).

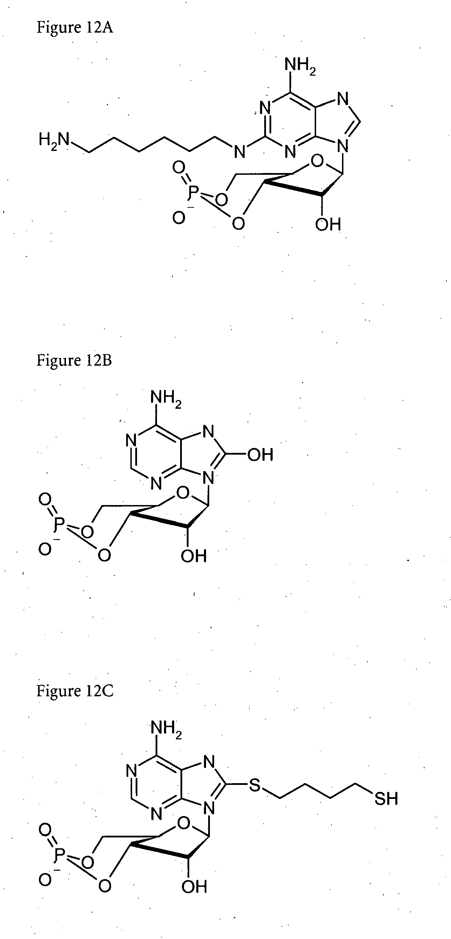

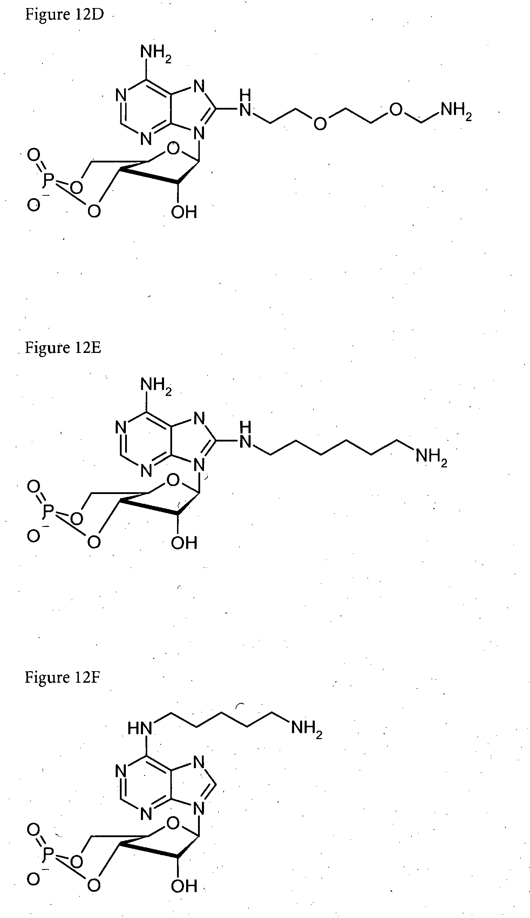

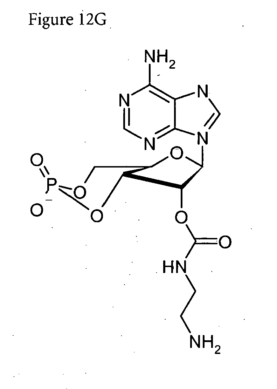

Figure 12 shows the chemical structures of cAMP derivates
   A): 2-(6-Aminohexyl)amino-cAMP (2-AHA-cAMP),
   B): 8-Hydroxy-cAMP (8-OH-cAMP)
   C): 8-(4-Mercaptobutylthio)-cAMP (8-MBT-cAMP)
   D): 8- (8- Amino- 3, 6- dioxaoctylamino) (8-ADOA-cAMP)
   E): 8-(6-Aminohexyl)amino-cAMP (8-AHA-cAMP)
   F): N-6-Aminohexyl-cAMP
   G): 2'-AEC-cAMP (AEC = 3-Amino-9-ethyl carbazole)

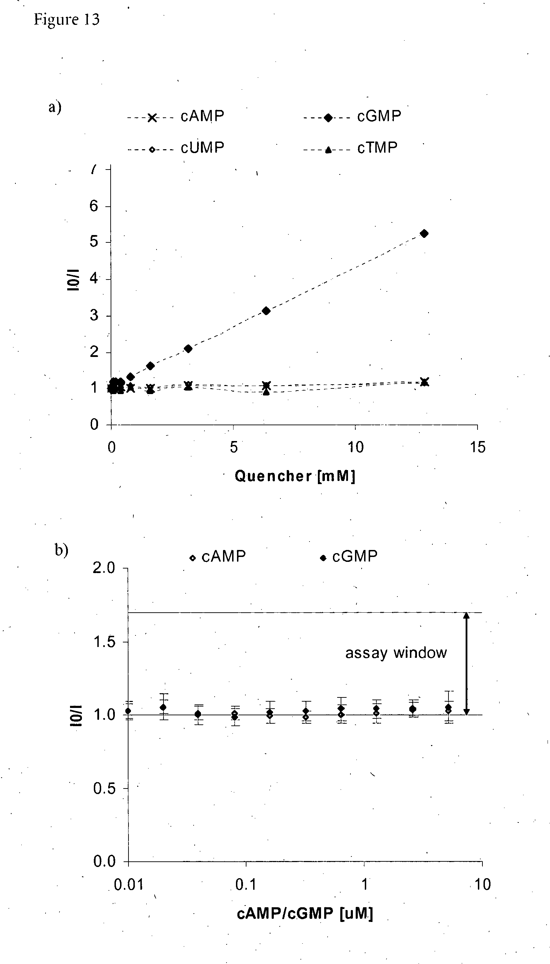

Figure 13 shows a graphical representation of the quenching of MR121 by cAMP, cGMP, cUMP and cTMP up to 13mM (a). (b) shows a graphical representation of the quenching of MR121 by cAMP and cGMP in the concentrations up to 10 µM in a Stern-Vollmer Plot. In low concentrations, neither cAMP nor cGMP quenches MR121.

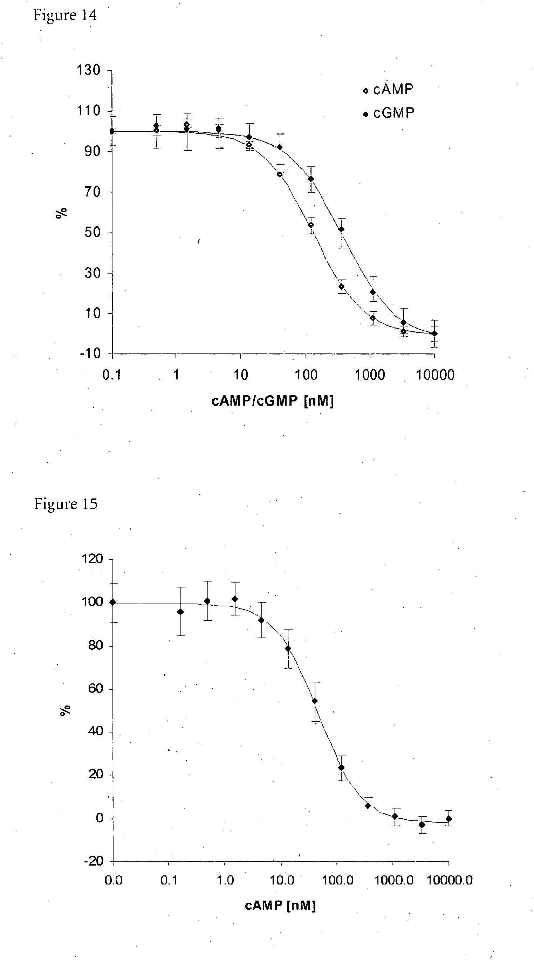

Figure 14 shows a graphical representation of the decrease of fluorescence in relation to the concentration of cAMP and cGMP (standard curves).
Figure 15 shows a graphical representation of the decrease of fluorescence in relation to the concentration of cAMP (cAMP standard curve) with Atto700-2-AHA-cAMP as tracer and anti-cAMP antibody as dequencher. Fitting of the curve gives IC50=48 nM.

### Examples

Commercially available reagents referred to in the examples were used according to manufacturer's instructions unless otherwise indicated.

### Reagents and Instrumentation

cAMP, cGMP, 2-AHA-cAMP, 8-AHA-cAMP, 8-ADOA-cAMP, 2'-AEC-cAMP, 8-MBT-cAMP, N-6-Aminohexyl-cAMP and 8-OH-cAMP were purchased from Biolog Life Science Institute (28071 Bremen, Germany), MR121 NHS ester and N-6-Aminohexyl-cAMP from Roche diagnostics (Penzberg, Germany) and Atto™ 700 NHS ester from Atto-Tec GmbH (Atto-Tec GmbH, Am Eichenhang 50, 57076 Siegen, Germany). Monoclonal mouse anti-cAMP antibodies were produced in-house as described by Kohler and Milstein (Nature 1975, 256: 495-497)

All experiments were performed in PBS (pH 7.4) containing 0.1% BSA (Albumin, bovine serum, ≥96%, essentially fatty acid free, A6003, Sigma-Aldrich Chemie GmbH, Industriestrasse 25, CH-9471 Buchs, Switzerland). To lyse the cells a 3x PBS lysis buffer (pH 7.4) was used containing 0.1% BSA (Albumine from bovine serum, fraction V, ≥96%, 05480, Sigma-Aldrich Chemie GmbH), 0.45% Triton^{®} X-100 (9342, Sigma-Aldrich Chemie GmbH), 0.075% NP40 (Nonidet P40 Substitute, 19628, USB Corporation, Cleveland, OH USA) and 0.3%o NaN₃ (purum p.a., >99%, 71290, Sigma-Aldrich Chemie GmbH). Growth medium for the cells was F-12K (Gibco 21127-002) with 10% FCS and 1% Penicillin-Streptomycin (Gibco 15140-122).

All fluorescence intensity measurements were carried out by means of a plate::vision fluorescence reader (Evotec Technologies GmbH, Schnackenburgallee 114, D-22525 Hamburg, Germany) equipped with a high pressure Xe arc lamp using for the measurements with MR121 an excitation filter at 630 nm (bandwidth 50 nm) and an emission filter at 695 nm (bandwidth 55 nm) and for the measurements with Atto 700 an excitation filter at 655 nm (bandwidth 50 nm) and an emission filter at 710 nm (bandwidth 40 nm). The fluorescent intensity was adjusted to about 60% of the maximal signal of the employed iCCD camera by using attenuation filters and varying exposure times. The lifetime measurements were performed by means of a plate::vision TRF reader (Evotec Technologies GmbH, Schnackenburgallee 114, D-22525 Hamburg, Germany) with an OPO system as light source (GWU Lasertechnik Vertriebsgesellschaft m.b.H., 50374 Erftstadt, Germany) tuned to 630 nm for excitation and the emission filter at 695 nm.

All experiments were done in 384 well microtiter plates (Costar 384, black with clear, flat bottom, tissue culture treated, Prod. No. 3712), the total assay volume varying from 30 to 40 µl.

### Example 1: Quenching of MR121

### 1.1 Quenching capabilities of cyclic nucleotides

20 µl MR121 (in PBS, end concentration 20 nM) and 20 µl quencher in PBS (DMSO end concentration in the assay: 1.25%) were pipetted into a microtiter plate. Fluorescence and lifetime were measured after an incubation time of 30 minutes at RT. The tested concentrations of the cyclic nucleotides were 0, 0.1, 0.2, 0.4, 0.8, 1.6, 3.2, 6.4 and 12.8 mM.

cGMP quenches MR121 at higher concentrations (Figure 13a). However, this quenching does not affect the assay window because such concentrations are not reached in a cGMP assay. In low concentrations up to 10µM (corresponds to range of cellular concentrations), neither cAMP nor cGMP quench the fluorophore (Figure 13b). In the displacement assay with the MR121-2-AHA-cAMP tracer the assay window for displacement with cAMP or cGMP goes from 1 (MR121-2-AHA-cAMP dequenched upon binding to the antibody) to 1.7 I/I₀ (MR121 quenched by 2-AHA-cAMP).

### 1.2 Quenching with cAMP derivates

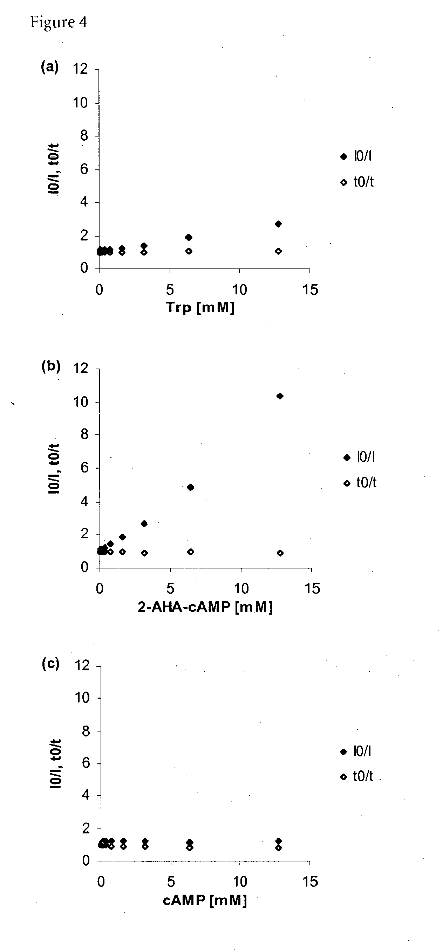

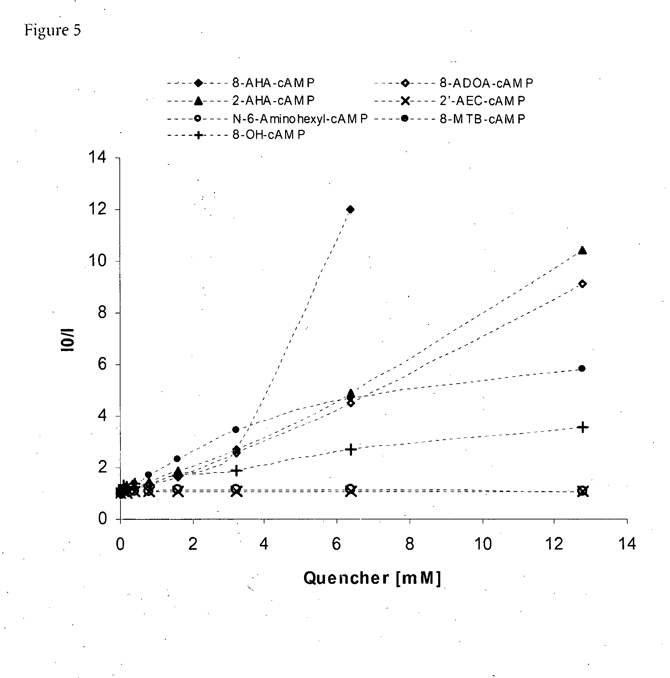

In order to analyze the quenching mechanism, measurements of fluorescence intensity and fluorescence lifetime of free MR121 as a function of different molecules (Trp, cAMP, 2-AHA-cAMP, 8-AHA-cAMP, 8-ADOA-cAMP, 2'-AEC-cAMP, N-6-Aminohexyl-cAMP, 8-MBT-cAMP and 8-OH-cAMP, see Figure 4, 5) were carried out.

20 µl MR121 (in PBS, end concentration 20 nM) and 20 µl quencher in PBS (DMSO end concentration in the assay: 1.25%) were pipetted into a microtiter plate. Fluorescence and lifetime were measured after an incubation time of 30 minutes at RT. A decrease in fluorescence intensity without a change in fluorescence lifetime is observed with Trp. Surprisingly the decrease of fluorescence intensity is even more pronounced for 2-AHA-cAMP, 8-AHA-cAMP, 8-ADOA-cAMP, 8-MBT-cAMP and 8-OH-cAMP (with constant fluorescence lifetime) while cAMP, 2'-AEC-cAMP and N-6-Aminohexyl-cAMP show no quenching at all. In analogy to references 2-5 we conclude that MR121 forms a non-fluorescent ground state complex with 2-AHA-cAMP, 8-AHA-cAMP, 8-ADOA-cAMP, 8-MBT-cAMP and 8-OH-cAMP but not with cAMP, 2'-AEC-cAMP and N-6-Aminohexyl-cAMP.

In 2-AHA-, 8-AHA-, and 8-ADOA-cAMP the linker is coupled via an amine bond, in 8-MBT-cAMP via a sulfur and in 8-OH-cAMP via an oxygen bond directly to the purine ring system of adenosine. However in N-6-Aminohexyl-cAMP and 2'-AEC-cAMP the linker is coupled either to the amine group or the ribose moiety of adenosine. Apparently the modification of the adenosine leads to a change in the electronic states of the ring system which then favors the complex formation with the oxazine dye.

In Figure 5 the change in fluorescence intensity (I₀/I) is plotted for all cAMP derivatives investigated. The association constant, Kₛ, was calculated from the linear region (up to 3.2 mM) of the plot giving 514 M⁻¹, 475 M⁻¹, 512 M⁻¹, 758 M⁻¹ and 236 M⁻¹ for 8-AHA-cAMP, 8-ADOA-cAMP, 2-AHA-cAMP, 8-MBT-cAMP and 8-OH-cAMP, respectively. This quenching is very efficient, the Kₛ for most of the cAMP derivatives is more than twice compared to tryptophane reported in the literature (∼220 M⁻¹).

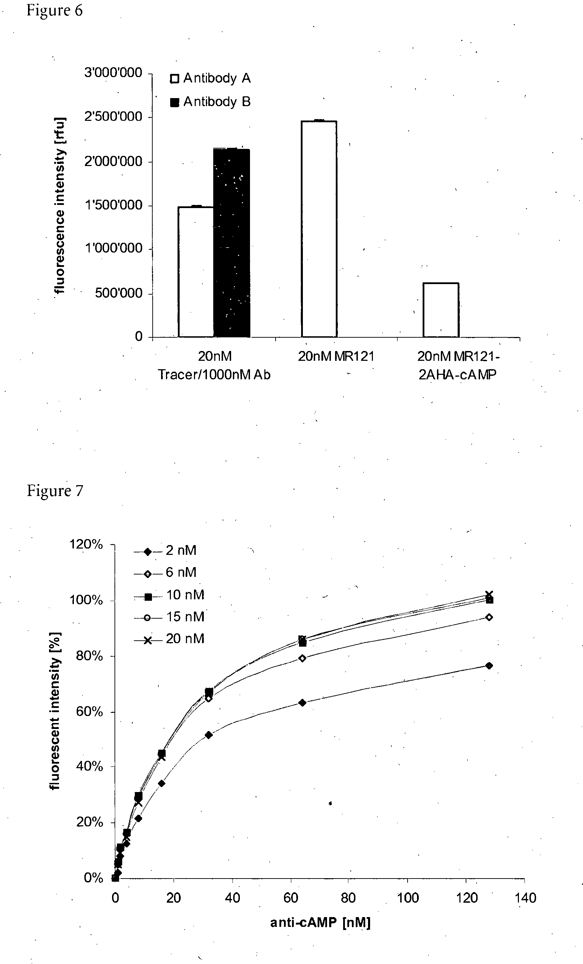

When MR121-2-AHA-cAMP is bound to a specific cAMP antibody this non-fluorescent complex cannot be formed and an increase in MR121 fluorescence intensity is observed (Figure 6). The fluorescence intensity of 20 nM free MR121, 20 nM MR121-2-AHA-cAMP (maximum quenching) and a mixture of 20 nM MR121-2-AHA-cAMP and 1000 nM antibody (maximum dequenching) was measured in PBS containing 0.1% BSA.

Compared to free MR121 (100%) the tracer shows a fluorescence intensity of 25%. Antibody A dequenches the fluorescence to 61%, antibody B to 87%. Apparently the degree of dequenching also depends on the binding affinity of the anti-cAMP antibody and on the amino acid sequence forming the binding domain of the antibody. A tryptophane in proximity to the binding domain of the antibody may quench the MR121 fluorescence to a certain degree. This can explain that different antibodies show different degrees of dequenching.

For the assay it is crucial that the antibody recognizes the tracer which is a labeled and modified cAMP and "pure" cAMP produced in the cells. For the further development of the assay we used antibody A although the dequenching is less compared to antibody B. However, the displacement of the tracer with cAMP is better with antibody A and the window full quenching - full dequenching is still good enough to develop a robust assay.

### Example 2: K_{d} determination of anti-cAMP antibody

The binding affinity of the anti-cAMP antibody (antibody A) to MR121-2-AHA-cAMP (K_{d}) was determined by titrating the antibody against several tracer concentrations (2, 6, 10, 15 and 20 nM) in 1x lysis buffer. 20 µl anti-cAMP dilutions were pipetted in 4 replicates into a 384 well microtiter plate. Then 20 µl MR121-2-AHA-cAMP were added to each well and the fluorescence intensity was read after 30 minutes incubation at RT on a 384 well shaker. The data were normalized by setting the values without antibody to 0%. Figure 7 shows the corresponding titration curves. Fitting to a four parameter model yields K_{d} values from 25 to 29 nM for the different tracer concentrations.

### Example 3: dose response curves

### cAMP

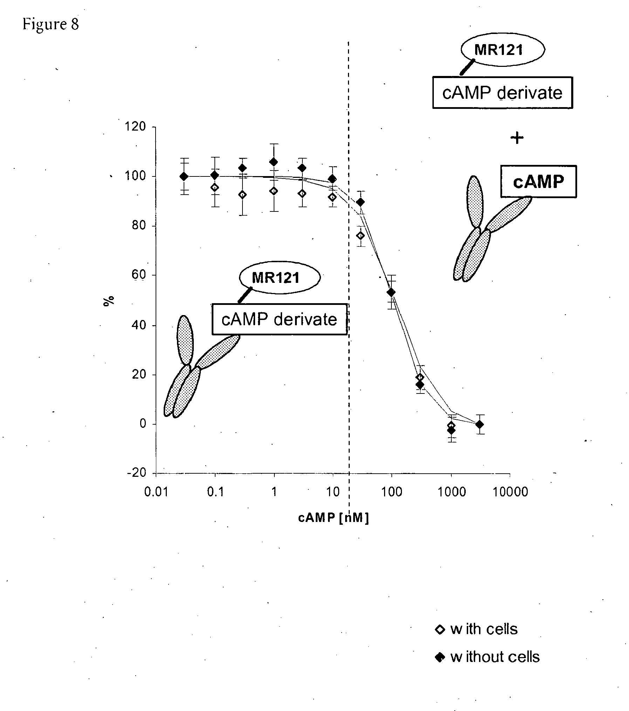

Based on K_{d}=28 nM 40 nM anti-cAMP antibody and 20 nM MR121-2-AHA-cAMP were chosen for a sensitive detection of cAMP with an IC₅₀ around 100 nM. Figure 8 displays two cAMP standard curves, one without cells and one with cells. For the standard curve without cells 20 µl cAMP dilutions in PBS containing 0.1% BSA were titrated into a 384 well microtiter plate and then 10 µl of the detection mix (120 nM anti-cAMP and 60 nM MR121-2-AHA-cAMP in 3x lysis buffer) were added. For the standard curve with cells 10'000 cells/well (CHO-K1) were plated in 25 µl medium and kept 20h at 37°C. The medium was then removed and 20 µl cAMP dilutions in PBS + 0.1% BSA followed by 10 µl detection mix were added. The plates were then incubated at RT on a 384 well shaker before reading the fluorescent signal. The determined IC₅₀'s are similar for both standard curves (109 and 119 nM without and with cells, respectively, see Figure 8).

The stability of the cAMP standard curve with respect to time and to tolerability against DMSO and BSA were tested without cells using the detection mix in lysis buffer. With up to 5% DMSO and 0.8% BSA no change in IC₅₀ and no significant drop of signal were observed. Signal and IC₅₀ are stable for at least 6h.

Figure 15 shows a cAMP dose response curve done with 5 nM Atto700-2-AHA-cAMP tracer and 5 nM antibody giving an IC₅₀ of 48 nM. cAMP was diluted in PBS containing 0.1% BSA. 20 µl of the cAMP dilutions were titrated into a 384 well microtiter plate and 10 µl detection mix were added. The plate was incubated at RT on a 384 well shaker before reading the fluorescent signal.

### cGMP

Figure 14 shows a comparison of the standard curves for cAMP and cGMP done with 20 nM MR121-2-AHA-cAMP tracer und 40 nM antibody giving an IC₅₀ of 134 nM for cAMP and 387 nM for cGMP. These IC₅₀ values as well as the sensitivity for cAMP and cGMP depend on the selection of the antibody. A different antibody may be more sensitive for cGMP and less sensitive for cAMP.

cAMP and cGMP were diluted in PBS containing 0.1% BSA. For the standard curve without cells 20 µl of cAMP dilutions and 20µl cGMP dilutions were titrated into a 384 well microtiter plate and then 10 µl of the detection mix (40 nM anti-cAMP and 20 nM MR121-2-AHA-cAMP in 3x lysis buffer) were added. The plates were then incubated at room temperature on a 384 well shaker before reading the fluorescent signal.

### Example 4: cAMP cellular assay

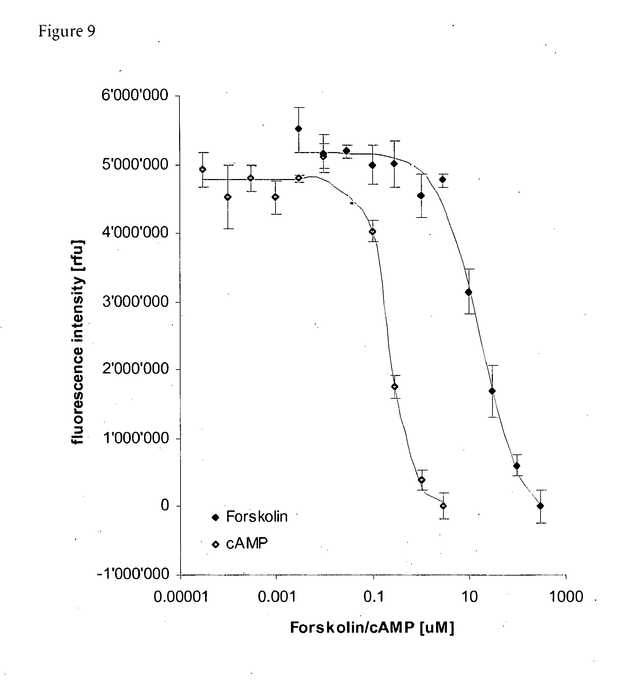

A forskolin dose response curve was performed with CHO-K1 cells. Cell numbers from 2'500 to 20'000 cell/well were plated in 25 µl medium and incubated for 20h at 37°C. The medium was removed and after adding 10 µl of PBS with 0.1% BSA and 1 mM IBMX the cells were incubated for 60 minutes at 37°C. 10 µl of forskolin dilutions in PBS + 0.1% BSA + 1 mM IBMX from 0.01 to 300 µM (final concentration in 20 µl assay volume) were added and incubated for another 30 minutes at 37°C. Then the cells were lysed by adding 10 µl detection mix (20 nM MR121-2-AHA-cAMP + 40 nM anti-cAMP, final concentration in 30 µl) in 3x lysis buffer and the fluorescence signal was read after 30 minutes incubation at RT on a 384 well shaker. With 10'000 cells/well the maximal cAMP level and minimal IC₅₀ for Forskolin (3.7 µM) was reached. Figure 9 shows a Forskolin dose response curve and a cAMP standard curve with 10'000 cells/well.

### Example 5: Quenching of ATTO™590 and ATTO™655

20 µl ATTO™***590*** and ATTO™***655*** respectively (in PBS, end concentration 20 nM) and 20 µl quencher (8-AHA-cAMP) in PBS (DMSO end concentration in the assay: 1.25%) were pipetted into a microtiter plate. Fluorescence and lifetime were measured after an incubation time of 30 minutes at RT. The results were incorporated in a Stern-Vollmer-Plot. The association constant, Kₛ, was calculated from the linear region (up to 3.2 mM) of the plot.

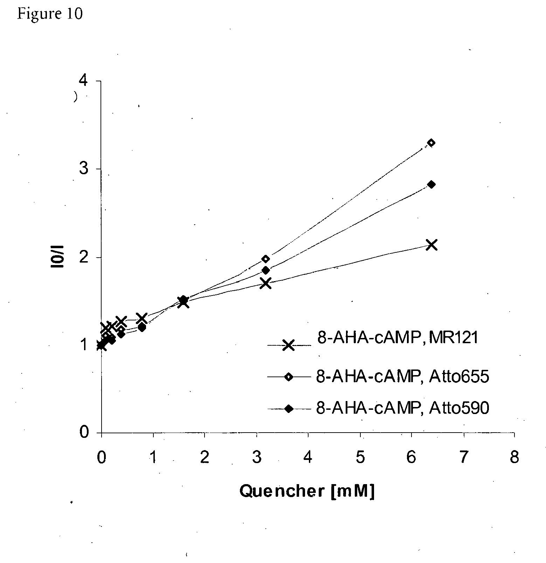

Atto™***590*** and Atto™***655*** are quenched by 8-AHA-cAMP with Kₛ=282 M⁻¹ and Kₛ=347 M⁻¹, respectively (Figure 10).

## Claims

1. In vitro method for detecting cyclic adenosine monophosphate (cAMP) or cyclic guanosine monophosphate (cGMP) comprising
a) contacting a mixture comprising cAMPor cGMP with a complex of a tracer and a dequencher, wherein the tracer is a fluorophore covalently linked to a cAMP quencher, the cAMP quencher is 2-(6-Aminohexyl)amino-cAMP, 8-(6-Aminohexyl)amino-cAMP, 8-(8- Amino- 3,6-dioxaoctylamino)-cAMP, 8-Hydroxy-cAMP or 8-(4-Mercaptobutylthio)-cAMP, and the cAMP quencher is able to quench the fluorophore it is covalently linked to, and the dequencher is able to bind the cAMP quencher as well as the cyclic nucleotide which shall be detected, and
b) measuring the change in fluorescence, wherein the measured change of the fluorescence is compared with a control.

2. The method according to claim 1, wherein the mixture is a cell lysate.

3. An in vitro method for determining the activity of a receptor wherein the signal transduction of the receptor comprises cAMP or cGMP
a) contacting cells expressing said receptor with a complex of a tracer and a dequencher, wherein the tracer is a fluorophore covalently linked to a cAMP quencher, the cAMP quencher is 2-(6-Aminohexyl)amino-cAMP, 8-(6-Aminohexyl)amino-cAMP, 8- (8-Amino- 3, 6- dioxaoctylamino)-cAMP, 8-Hydroxy-cAMP or 8-(4-Mercaptobutylthio)-cAMP, and the cAMP quencher is able to quench the fluorophore it is covalently linked to, and the dequencher is able to bind the cAMP quencher as well as the cyclic nucleotide which shall be detected,
b) lysing the cells, and
c) measuring the change in fluorescence, wherein the measured change of the fluorescence is compared with a control.

4. The method according to claim 3, wherein step b) is prior of step a).

5. An in vitro method for screening of a ligand for a receptor wherein the signal transduction of the receptor comprises cAMP or cGMP comprising
a) contacting a candidate compound with a cell expressing said receptor
b) adding to the cells a complex of a tracer and a dequencher, wherein the tracer is a fluorophore covalently linked to a cAMP quencher, the cAMP quencher is 2-(6-Aminohexyl)amino-cAMP, 8-(6-Aminohexyl)amino-cAMP, 8- (8- Amino- 3, 6-dioxaoctylamino)-cAMP, 8-Hydroxy-cAMP or 8-(4-Mercaptobutylthio)-cAMP, and the cAMP quencher is able to quench the fluorophore it is covalently linked to, and the dequencher is able to bind the cAMP quencher as well as the cyclic nucleotide which shall be detected,
c) lysing cells, and
d) measuring the change in fluorescence, wherein the measured change of the fluorescence is compared with a control.

6. The method according to claim 5, wherein step c) is prior of step b).

7. The method according to any one of claims 3 to 6 wherein the receptor is a G-protein-coupled receptor.

8. The method according to any one of claims 1 to 7, wherein the cAMP quencher is 2-(6-Aminohexyl)amino-cAMP.

9. The method according to any one of the claims 1 to 8, wherein the fluorophore is 1-[3-(2,5-Dioxo-pyrrolidin-1-yloxycarbonyl)-propyl]-11-ethyl-3,4,8,9,10,11-hexahydro-2H-13-oxa-6,11-diaza-1-azonia-pentacene (MR121).

10. Use of the method according to any one of the claims 1, 2, 8 and 9 for determining cAMP or cGMP concentration in a mixture.

11. A kit comprising a tracer and a dequencher, wherein the tracer is a fluorophore covalently linked to a cAMP quencher, the cAMP quencher is 2-(6-Aminohexyl)amino-cAMP, 8-(6-Aminohexyl)amino-cAMP, 8- (8- Amino- 3, 6- dioxaoctylamino)-cAMP, 8-Hydroxy-cAMP or 8-(4-Mercaptobutylthio)-cAMP, and the cAMP quencher is able to quench the fluorophore it is covalently linked to, and the dequencher is able to bind the cAMP quencher as well as the cyclic nucleotide which shall be detected.

## Patentansprüche

1. In vitro Verfahren zur Detektion von cyclischem Adenosinmonophosphat (cAMP) oder cyclischem Guanosinmonophosphat (cGMP) umfassend
a) Inkontaktbringen eines Gemisches umfassend cAMP oder cGMP mit einem Komplex eines Tracers und eines Dequenchers, wobei der Tracer ein, mit einem cAMP-Quencher kovalent verbundenes Fluorophor ist, der cAMP-Quencher 2-(6-Aminohexyl)amino-cAMP, 8-(6-Aminohexyl)amino-cAMP, 8-(8-Amino-3,6-dioxaoctylamino)-cAMP, 8-Hydroxy-cAMP oder 8-(4-Mercaptobutylthio)-cAMP ist und der cAMP-Quencher fähig ist das Fluorophor, an welches er kovalent gebundenen ist, zu quenchen und der Dequencher fähig ist den cAMP-Quen-cher, sowie das cyclische Nukleotid, welches detektiert werden soll, zu binden, und
b) Messen der Fluoreszenzänderung, wobei die gemessene Fluoreszenzänderung mit einer Kontrolle verglichen wird.

2. Verfahren nach Anspruch 1, wobei das Gemisch ein Zelllysat ist.

3. In vitro Verfahren zur Bestimmung der Aktivität eines Rezeptors, wobei die Signaltransduktion des Rezeptors cAMP oder cGMP beinhaltet
a) Inkontaktbringen von Zellen, die den Rezeptor exprimieren mit einem Komplex eines Tracers und eines Dequenchers, wobei der Tracer ein, mit einem cAMP-Quencher kovalent verbundenes Fluorophor ist, der cAMP-Quencher 2-(6-Aminohexyl)amino-cAMP, 8-(6-Aminohexyl)amino-cAMP, 8-(8-Amino-3,6-dioxaoctylamino)-cAMP, 8-Hydroxy-cAMP oder 8-(4-Mercaptobutylthio)-cAMP ist und der cAMP-Quencher fähig ist das Fluorophor, an welches er kovalent gebundenen ist, zu quenchen und der Dequencher fähig ist den cAMP-Quencher, sowie das cyclische Nukleotid, welches detektiert werden soll, zu binden,
b) Lysieren der Zellen, und
c) Messen der Fluoreszenzänderung, wobei die gemessene Fluoreszenzänderung mit einer Kontrolle verglichen wird.

4. Verfahren nach Anspruch 3, wobei Schritt b) vor Schritt a) erfolgt.

5. In vitro Verfahren zum Screenen eines Liganden für einen Rezeptor, wobei die Signaltransduktion des Rezeptors cAMP oder cGMP beinhaltet, umfassend
a) Inkontaktbringen einer Kandidatenverbindung mit einer Zelle, die den Rezeptor exprimiert,
b) Zugeben eines Komplexes eines Tracers und eines Dequenchers zu den Zellen, wobei der Tracer ein, mit einem cAMP-Quencher kovalent verbundenes Fluorophor ist, der cAMP-Quencher 2-(6-Aminohexyl)amino-cAMP, 8-(6-Aminohexyl)amino-cAMP, 8-(8-Amino-3,6-dioxaoctylamino)-cAMP, 8-Hydroxy-cAMP oder 8-(4-Mercaptobutylthio)-cAMP ist und der cAMP-Quencher fähig ist das Fluorophor, an welches er kovalent gebundenen ist, zu quenchen und der Dequencher fähig ist den cAMP-Quencher, sowie das cyclische Nukleotid, welches detektiert werden soll, zu binden,
c) Lysieren der Zellen, und
d) Messen der Fluoreszenzänderung, wobei die gemessene Fluoreszenzänderung mit einer Kontrolle verglichen wird.

6. Verfahren nach Anspruch 5, wobei Schritt c) vor Schritt b) erfolgt.

7. Verfahren nach einem der Ansprüche 3 bis 6, wobei der Rezeptor ein G-Protein-gekoppelter Rezeptor ist.

8. Verfahren nach einem der Ansprüche 1 bis 7, wobei der cAMP-Quencher 2-(6-Aminohexyl)amino-cAMP ist.

9. Verfahren nach einem der Ansprüche 1 bis 8, wobei das Fluorophore 1-[3-(2,5-Dioxo-pyrrolidin-1-yloxycarbonyl)-propyl]-11-ethyl-3,4,8,9,10,11-hexahydro-2H-13-oxa-6,11-diaza-1-azonia-pentacen (MR121) ist.

10. Verwendung des Verfahrens nach einem der Ansprüche 1, 2, 8 oder 9 zum Bestimmen der cAMP oder cGMP Konzentration in einem Gemisch.

11. Kit umfassend einen Tracer und einen Dequencher, wobei der Tracer ein, mit einem cAMP-Quencher kovalent verbundenes Fluorophor ist, der cAMP-Quencher 2-(6-Aminohexyl)amino-cAMP, 8-(6-Aminohexyl)amino-cAMP, 8-(8-Amino-3,6-dioxaoctylamino)-cAMP, 8-Hydroxy-cAMP oder 8-(4-Mercaptobutylthio)-cAMP ist und der cAMP-Quencher fähig ist das Fluorophor, an welches er kovalent gebundenen ist, zu quenchen und der Dequencher fähig ist den cAMP-Quen-cher, sowie das cyclische Nukleotid, welches detektiert werden soll, zu binden.

## Revendications

1. Procédé *in vitro* de détection de monophosphate d'adénosine cyclique (cAMP) ou de monophosphate de guanosine cyclique (cGMP) comprenant
a) la mise en contact d'un mélange comprenant du cAMP ou du cGMP avec un complexe d'un traceur et d'un réactivateur, où le traceur est un fluorophore lié par covalence à un désactivateur de type cAMP, le désactivateur de type cAMP est le 2-(6-aminohexyl)amino-cAMP, le 8-(6-aminohexyl)amino-cAMP, le 8-(8-amino-3,6-dioxaoctylamino)-cAMP, le 8-hydroxy-cAMP ou le 8-(4-mercaptobutylthio)-cAMP, et le désactivateur de type cAMP est capable de désactiver le fluorophore auquel il est lié par covalence, et le réactivateur est capable de se lier au désactivateur de type cAMP ainsi qu'au nucléotide cyclique qui doit être détecté, et
b) la mesure du changement de fluorescence, le changement mesuré de la fluorescence étant comparé à un témoin.

2. Procédé selon la revendication 1, où le mélange est un lysat de cellules.

3. Procédé *in vitro* de détection de l'activité d'un récepteur, où la transduction du signal du récepteur comprend le cAMP ou le cGMP, comprenant
a) la mise en contact de cellules exprimant ledit récepteur avec un complexe d'un traceur et d'un réactivateur, où le traceur est un fluorophore lié par covalence à un désactivateur de type cAMP, le désactivateur de type cAMP est le 2-(6-aminohexyl)amino-cAMP, le 8-(6-aminohexyl)amino-cAMP, le 8-(8-amino-3,6-dioxaoctylamino)-cAMP, le 8-hydroxy-cAMP ou le 8-(4-mercaptobutylthio)-cAMP, et le désactivateur de type cAMP est capable de désactiver le fluorophore auquel il est lié par covalence, et le réactivateur est capable de se lier au désactivateur de type cAMP ainsi qu'au nucléotide cyclique qui doit être détecté,
b) la lyse des cellules, et
c) la mesure du changement de fluorescence, le changement mesuré de la fluorescence étant comparé à un témoin.

4. Procédé selon la revendication 3, où l'étape b) se situe avant l'étape a).

5. Procédé *in vitro* de criblage d'un ligand pour un récepteur, où la transduction du signal du récepteur comprend le cAMP ou le cGMP, comprenant
a) la mise en contact d'un composé candidat avec une cellule exprimant ledit récepteur,
b) l'addition aux cellules d'un complexe d'un traceur et d'un réactivateur, où le traceur est un fluorophore lié par covalence à un désactivateur de type cAMP, le désactivateur de type cAMP est le 2-(6-aminohexyl)amino-cAMP, le 8-(6-aminohexyl)amino-cAMP, le 8-(8-amino-3,6-dioxaoctylamino)-cAMP, le 8-hydroxy-cAMP ou le 8-(4-mercaptobutylthio)-cAMP, et le désactivateur de type cAMP est capable de désactiver le fluorophore auquel il est lié par covalence, et le réactivateur est capable de se lier au désactivateur de type cAMP ainsi qu'au nucléotide cyclique qui doit être détecté,
c) la lyse des cellules, et
d) la mesure du changement de fluorescence, le changement mesuré de la fluorescence étant comparé à un témoin.

6. Procédé selon la revendication 5, où l'étape c) se situe avant l'étape b).

7. Procédé selon l'une quelconque des revendications 3 à 6, où le récepteur est un récepteur couplé à la protéine G.

8. Procédé selon l'une quelconque des revendications 1 à 7, où le désactivateur de type cAMP est le 2-(6-aminohexyl)amino-cAMP.

9. Procédé selon l'une quelconque des revendications 1 à 8, où le fluorophore est le 1-[3-(2,5-dioxopyrrolidin-1-yloxycarbonyl)propyl]-11-éthyl-3,4,8,9,10,11-hexahydro-2H-13-oxa-6,11-diaza-1-azoniapentacène (MR121).

10. Utilisation du procédé selon l'une quelconque des revendications 1, 2, 8 et 9 pour la détermination de la concentration de cAMP ou de cGMP dans un mélange.

11. Kit comprenant un traceur et un réactivateur, où le traceur est un fluorophore lié par covalence à un désactivateur de type cAMP, le désactivateur de type cAMP est le 2-(6-aminohexyl)amino-cAMP, le 8-(6-aminohexyl)amino-cAMP, le 8-(8-amino-3,6-dioxaoctylamino)-cAMP, le 8-hydroxy-cAMP ou le 8-(4-mercaptobutylthio)-cAMP, et le désactivateur de type cAMP est capable de désactiver le fluorophore auquel il est lié par covalence, et le réactivateur est capable de se lier au désactivateur de type cAMP ainsi qu'au nucléotide cyclique qui doit être détecté.
